(19) **Europäisches Patentamt European Patent Office Office européen des brevets**

(11) **EP 3 181 097 B1**

(12) **EUROPEAN PATENT SPECIFICATION**

(45) Date of publication and mention
of the grant of the patent:
**07.08.2019 Bulletin 2019/32**

(51) Int Cl.:
**A61F 2/28** (2006.01)     **A61L 27/56** (2006.01)

(21) Application number: **15832600.9**

(22) Date of filing: **23.06.2015**

(86) International application number:
**PCT/CN2015/082066**

(87) International publication number:
**WO 2016/023403 (18.02.2016 Gazette 2016/07)**

(54) **LOW-MODULUS MEDICAL IMPLANTATION POROUS SUPPORT STRUCTURE**

PORÖSE STÜTZSTRUKTUR FÜR MEDIZINISCHES IMPLANTAT MIT GERINGEM MODULUS

STRUCTURE DE SUPPORT PERFORÉE POUR IMPLANT À USAGE MÉDICAL À FAIBLE MODULE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.08.2014 CN 201410401502**

(43) Date of publication of application:
**21.06.2017 Bulletin 2017/25**

(73) Proprietor: **Fujian Institute Of Research On The Structure Of
Matter, Chinese Academy Of Sciences**
**Fuzhou, Fujian 350002 (CN)**

(72) Inventors:
• **LIN, Jinxin**
  **Fuzhou**
  **Fujian 350002 (CN)**
• **WU, Songquan**
  **Fuzhou, 350002 (CN)**
• **LIN, Junjie**
  **Fuzhou**
  **Fujian 350002 (CN)**
• **LU, Yanjin**
  **Fuzhou**
  **Fujian 350002 (CN)**
• **GAN, Yiliang**
  **Fuzhou**
  **Fujian 350002 (CN)**
• **ZHAO, Chaoqian**
  **Fuzhou**
  **Fujian 350002 (CN)**

(74) Representative: **Rottenberg, Annabell Simone
Tropa ApS**
**Aagade 97, 1st Floor**
**8370 Hadsten (DK)**

(56) References cited:
| | |
|---|---|
| WO-A1-2009/048314 | CN-A- 102 548 509 |
| CN-A- 103 445 883 | CN-A- 103 445 883 |
| CN-A- 104 207 867 | CN-U- 204 033 546 |
| CN-Y- 201 200 499 | US-A1- 2004 010 315 |
| US-A1- 2011 014 081 | US-A1- 2013 123 935 |
| US-A1- 2013 178 947 | US-A1- 2013 218 288 |

## Description

## TEHCHICAL FIELD

The present disclosure relates to a porous scaffold structure, particularly to a medical implant porous scaffold structure having low modulus, belonging to the field of medical implant materials.

## BACKGROUND OF THE INVENTION

In the field of the treatment and repair of bone diseases such as bone fracture or bone necrosis, particularly in the treatment and repair of the load-bearing bone, surgery, in which a dead bone is replaced with an implant, is a common and useful way to prevent bone diseases from further deteriorating and to avoid late fractures and even disability. So far, common implants mainly include autologous bone, allogenic bone, bioceramic, organic polymer, degradable materials, metal materials and so on. However, the autologous bone transplantation may cause donor site pain and has limited sources, the allogenic bone transplantation has the possibility of immune reaction and viral infection, the bioceramic has intrinsic brittleness, the organic polymer has a too low strength, and the degradable materials are still in the stage of laboratory research. Accordingly, neither of these materials has not been widely used in the treatment and repair of the load-bearing bone. On the contrary, metallic materials such as stainless steel, Co-Cr based alloys, titanium based alloys have been widely applied in the clinic for their good mechanical properties, together with corrosion resistance, biocompatibility and so on.

However, the metallic materials have a modulus (stainless steel: about 200 GPa, Co-Cr based alloy: about 230 GPa, titanium based alloys: 50 to 110 GPa) significantly higher than that of the bone tissue (the modulus range of the cortical bone: about 2 to 25 GPa). Therefore, they may cause the so-called "stress shielding effect", namely the effect that stress mainly transfers through the metal implants. At this time, the bone tissue surrounding the metal implants will withstand a low-load state for a long term, leading to osteoporosis caused by bone resorption, in which case the implant would easily loose, and the bone tissue is prone to fracture under stress. Currently, the "stress shielding effect" as an important reason for shortening the service time of the metal implant, can invisibly increase the replacement frequency of the implants of the patients, and meanwhile aggravate the pain of the patients and prolong the treatment.

As to the problem of "stress shielding" occurs in the treatment of bone diseases, the first problem to be solved is the modulus problem of the implant per se, which requires the modulus of the implant to be reduced to the degree matching the modulus of the bone tissue. In prior art, the materials are prepared into a porous shape to reduce the apparent modulus of the whole materials. However, by doing so, the properties such as strength and plasticity of the materials would also decline considerably accompanied by the increase of the porosity. Thus such materials prepared according to the prior art can hardly achieve an ideal match between low modulus and high strength. Moreover, a study found that the porous structure has a high permeability and therefore an implant having the porous structure may facilitate bone cell adhesion, nutrient transfer and the in-growth of new bone tissue and the like. However, these porous metal materials disclosed in the prior art possess pore size and distribution in random manner, differing from the bone tissue morphology of human oriented growth. At the same time, the isotropic pores distributed randomly not only provide no support the in-growth of bone cells and tissue, but also possess no distinction of stress distribution between the directions of high and low stress. In addition, these porous metal materials also have complex production processes that possess internal defects and may dope substances detrimental to human health. A porous scaffold structure formed from hexagonal prisms is disclosed WO-A-2009048314.

## DETAILED DESCRIPTION OF THE INVENTION

In order to overcome the problems of the above prior art structures in bone defects or necrosis treatment, the present disclosure provides a medical implant porous scaffold structure having low modulus. The structure could reduce the modulus of the implant, achieve an ideal match between the modulus and the strength of the implant, and improve the configuration of traditional metal implants to optimize their mechanical distribution, decrease the stress shielding effect. Furthermore, it has a regular interconnected pores structure, which can be conducive to bone tissue in-growth, increase mutual locking between bone tissue and implant, and shorten the recovery time of patients.

The technical solution according to the present disclosure is described as follows.

A medical implant porous scaffold structure having low modulus, wherein said structure is formed by multiple basic units superposed sequentially along the three-dimensional directions in three-dimensional space, each of the basic units is composed of a quadrangular prism or hexagonal prism having central interconnected pores encircled by four or six side walls, each of the side walls is composed by a "X-type" frame structure formed by two crossed ribs, and the central interconnected pores of the adjacent basic units arranged along the axis direction of the quadrangular prism or the hexagonal prism are interconnected to each other, the ratio of the equivalent circle diameter of the cross section of the rib to the length of the rib is 0.1 to 0.5; the ratio of the height of the quadrangular prism or the hexagonal prism to the base length of the side wall of the quadrangular prism or the hexagonal prism is 1.0 to 2.5.

First, as to the treatment of the load-bearing bone defect, the implant using the scaffold structure has a modulus matching the bone tissue so that the "stress shielding effect" prone to occur during treatment can be reduced or avoided, and the materials have a strength sufficient for mechanical support.

Secondly, due to its high porosity and permeability, the scaffold structure, when being used in the implant, may facilitate the bone cell adhesion, nutrient transfer and in-growth of new bone tissue.

Third, the quadrangular prisms or hexagonal prisms having central interconnected pores are vertically stacked, so that the central interconnected pores form up-down connected hollow catheters having orientation. This may facilitate the osteoblasts, chondroblasts, fibroblasts and capillaries to develop from the proliferation along the direction of the catheter to osteogenic differentiation, and thus is advantageous for bone healing.

The rib of the prism may have a cross section in the shape of solid circle, solid oval or solid polygon, so as to meet the various needs of the implants under different conditions. Alternatively, the rib may also have a cross section in the shape of hollow circular ring, hollow oval ring or hollow polygonal ring. Under the condition that the boundary condition and loading are the same and require the same strength, as compared to the solid prism, the hollow prism can use less materials and therefore save the materials, reduce the dead weight, and further reduce the modulus.

The central interconnected pore can have a cross section with the inscribed circle radius of 150 $\mu$m-750 $\mu$m, which may favor the in-growth of the osteoblasts, chondroblasts and bone tissue, enhance the mutual locking between the bone tissue and the scaffold. In case that the inscribed circle radius is too small, for example less than 150 $\mu$m, the in-growth of the osteoblasts, chondroblasts and bone tissue would be not easy. On the contrary, in case that the inscribed circle radius is too big, for example greater than 750 $\mu$m, osteoblasts, chondroblasts and bone tissue is not easy to load on the inner wall of the hollow catheters, which is formed by the up-down connection of the central interconnected pores, and thus is prone to fall off. Further, the inscribed circle radius of the cross section of said central interconnected pore in the scaffold structure can be determined by the parameters of the internal structure, rather than changes in isolation. The inscribed circle radius and the parameters have the following relationship:

for the hexagonal prism:

$$r = 0.866a\text{-}t$$

for the quadrangular prism:

$$r = 0.5a\text{-}t$$

wherein r is the inscribed circle radius of the cross section of the central interconnected pore, $t$ is the thickness of the rib or the equivalent circle diameter of the cross section of the rib, and $a$ is the base length of the quadrangular prism or hexagonal prism.

By selecting different materials (Ta: 183 GPa, Zr: 99 GPa, $\alpha$-Ti: 110 GPa, medical 316 stainless steel: 193 GPa, hydroxyapatite: 165 GPa, Co-Cr-Mo: 248 GPa, Co-Cr-W: 232 GPa, Co-base: 194 GPa, Mg: 44 GPa), the relationship between the overall modulus of the scaffold and the intrinsic modulus and structural parameters of the scaffold materials were simulated according to the above scheme. It is found that with a certain structure, the overall modulus of different scaffolds are proportional to the intrinsic modulus of the scaffold materials; with a certain material, as the ratio of the equivalent circle diameter of the cross section of the rib in the structure increases, the overall modulus of the scaffolds would increase therewith; with a certain material, as the ratio of the height of the quadrangular prism or the hexagonal prism to the base length of the side wall of the quadrangular prism or the hexagonal prism, the overall modulus of the scaffolds would increase therewith. In order to make the overall modulus of different scaffolds in the required range of 2 to 30 GPa, the ratio of the equivalent circle diameter of the cross section of the rib to the length of rib is 0.1 to 0.5; the ratio of the height of the quadrangular prism or the hexagonal prism to the base length of the side wall of the quadrangular prism or the hexagonal prism is 1.0 to 2.5.

Due to the bone tissue guide or induction of the implant after implantation in the vertical direction, vascular tissue generation and nutrient transfer needed in the vertical direction would be greater than those needed in the horizontal direction. Therefore, the cross section area of the central interconnected pore is preferably greater than the cross section area of the pore of the "X-type" framework structure in the sidewall.

The principles of the present medical implant porous scaffold structure having low modulus provided herein are described as follows.

(1) Modulus control

For solid materials, the modulus are generally belonging to the property that is insensitive to the structure and thus is very difficult to be significantly changed by adjusting the components, process or organization. For the porous materials, however, the overall apparent modulus can be further adjusted by the variation of porosity. Nevertheless, such adjustment would often greatly sacrifice the strength of materials, which may often cause the high porosity materials to be unable to meet the requirements of high strength. With the appearance of 3D printing technology, the pore structure and size of materials can be controlled precisely. As compared to traditional methods, 3D printing technology actually provides the porous materials with more control factors, which means that the modulus and strength of materials can be controlled by designing the pore parameters, so that achieve an ideal matching between the modulus and strength of the materials.

For the treatment of bearing bone defects, the requirements include not only the implants having a modulus matching that of the bone tissues to decrease or avoid the "stress shielding effect" prone to occur in the treatment process, but also the materials having sufficient strength to provide mechanical support. Thus, a porous implant generally should meet the both design requirements of the low modulus and high strength at the same time. The present disclosure selected the "X-type" structure as the basic framework to stack the three-dimensional porous scaffold structure, and achieved precise control of the modulus of the scaffold while achieving adequate mechanical support through the control of the support parameters.

(2) Interconnected pore design

It has been found in the morphology observation of bone section experiment that, the bone tissue is a porous material having clear orientation, wherein the porous framework served as the mechanical support, and the oriented pores may facilitate the nutrient transfer and promote the bone metabolism. By imitating the pore state features of the bone tissue, the present disclosure used the structure design of the interconnected pores in the implant scaffold. Specifically, the central interconnected pores units of the hexagonal prism or the quadrangular prism were encircled by the "X-type" frames. Afterwards, these units were periodically stacked in space to obtain porous scaffolds with regularly arranged hexagonal or quadrilateral interconnecting pores, so as to facilitate the substances transfer, bone tissue guide or induction, vascularization and other biological functions of the implants after their implantation.

(3) Structure selection

According to the different mechanics properties of the internal ribs of the porous scaffold materials, the scaffold structure can be mainly divided into tensile dominant structure (under external load, the ribs mainly show tensile and compressive deformation modes to resist external loading and whole deformation) and bending dominated structure (under the external load, the ribs mainly shows bending deformation mode to resist external loading and whole deformation). Due to the different stress modes of their internal edge ribs, the two types of structural materials have a great difference in terms of specific stiffness and specific strength. Tensile dominant structure is strong and brittle, and bending dominated structure is soft and tough.

According to the performance requirements of bone implant materials, actually neither of the above two types of structures can completely meet the needs. Colligating the two types of structures, the "X-type" framework structure was selected by this invention, which could increase the deformation capacity of the supporting materials as far as possible without excessively reducing the strength of the scaffold structure materials.

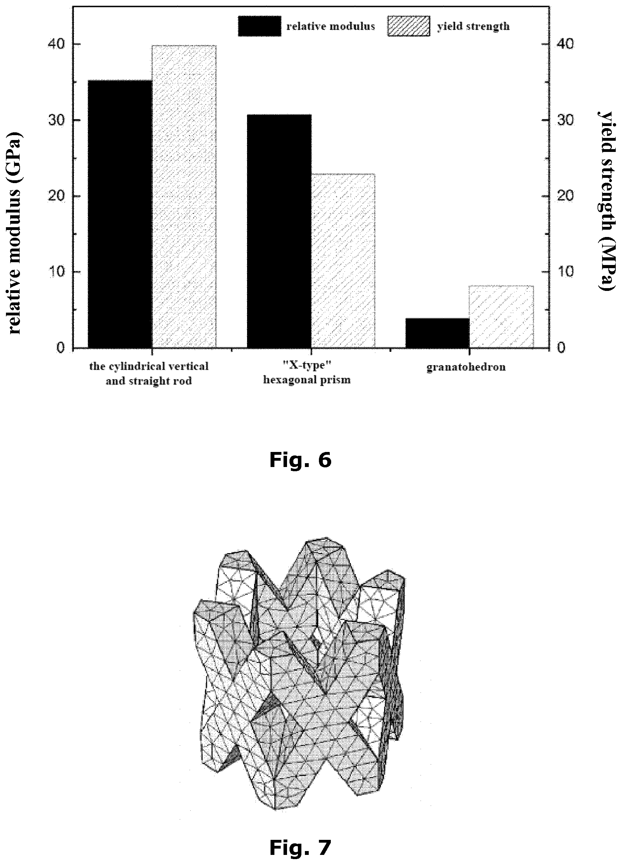

When α-Ti (E=110 GPa, ν=0.33) was selected as the implant material, as shown in Figure 6, the cylindrical vertical and straight rods with the same relative density (43.4%) were selected as the tensile dominant structure model, and the granatohedron was selected as the bending dominant structure model. As compared to the "X-type" hexagonal prism, it can be learned that the modulus and the strength of the "X-type" framework structure respectively fall between those of the tensile dominant structure and the bending dominated structure. Experiments with different implant materials (Ta: 183 GPa, Zr: 99 GPa, medical 316 stainless steel: 193 GPa, hydroxyapatite: 165 GPa, Co-Cr-Mo: 248 GPa, Co-Cr-W: 232 GPa, Co-based: 194 GPa, Mg: 44 GPa) showed that, "X-type" framework structure could increase the deformation capacity of the supporting materials as far as possible without excessively reducing the strength of the scaffold structure materials.

In addition, in view of the requirements of "modulus control" and "interconnected pore design", finally, the elected porous scaffold structure used the "X-type" framework as the side walls to encircle the hexagonal prism or the quadrangular prism structure unit, and then was form by the periodic translation of the structural unit in the space.

The medical implant porous scaffold structure having low modulus provided herein has the following advantages:

1) The materials with the structure have high porosity, large specific surface area, excellent impact energy absorption property and could provide adequate strength to provide mechanical support on the occasion of high strength requirements.

2) The pores with orientation may facilitate the nutrient transfer and promote the bone metabolism, and may facilitate substances transfer, bone tissue guide or induction, vascularization and other biological functions of the implants after their implantation.

3) The size or structure of the pores and the surface treatment of the scaffold structure according to different human bones and biological environments.

4) Achieving the ideal matching between the modulus and strength of the implant to reduce or even eliminate the "stress shielding effect" caused by the modulus difference between the solid metal and bone tissue, thereby increasing the service life of the implant and relieving the pain of patients.

5) The regular interconnected pore structure is beneficial to bone in-growth, increase the mutual locking between bone tissue and support materials, and reduce the recovery time of patients.

**DESCRIPTION OF THE DRAWING**

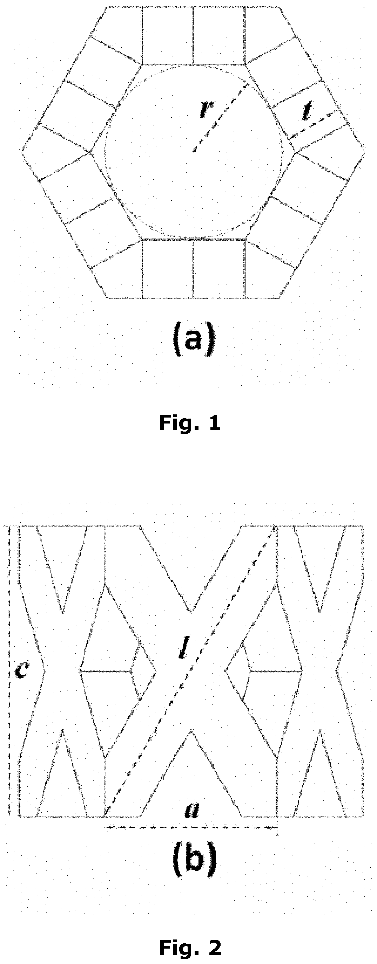

Figure 1    is the top view of the basic unit of the hexagonal prism according to the present disclosure.

Figure 2    is the side view of the basic unit of the hexagonal prism according to the present disclosure.

Figure 3    is the top view of the basic unit of the quadrangular prism according to the present disclosure.

Figure 4    is the side view of the basic unit of the quadrangular prism according to the present disclosure.

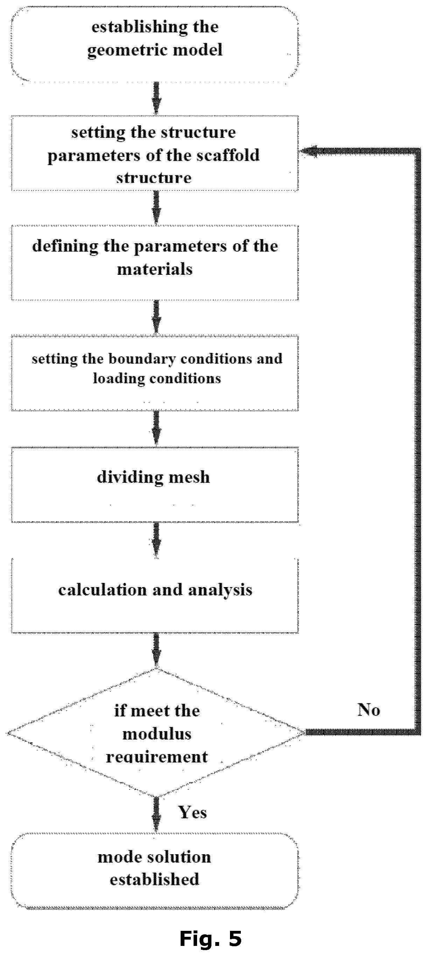

Figure 5    is the finite element analysis flow chart according to the present disclosure.

Figure 6    is a bar graph of the relative modulus and yield strength of the cylindrical vertical and straight rod structure, "X type" hexagonal prism, granatohedron structure according to the present disclosure.

Figure 7    is the finite element mesh division of the basic unit of the hexagonal prism according to the present disclosure.

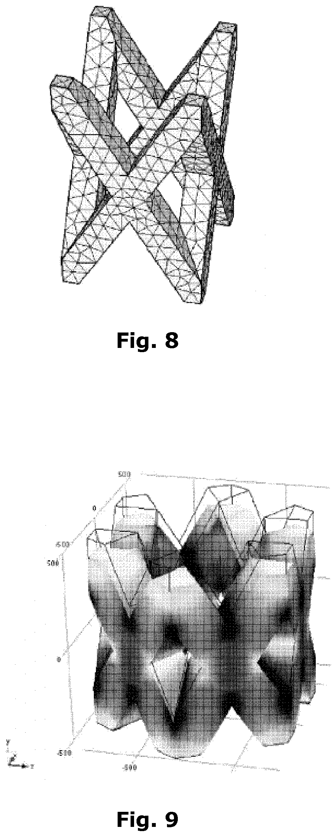

Figure 8    is the finite element mesh division of the basic unit of the quadrangular prism according to the present disclosure.

Figure 9    is the finite element simulation analysis process chart of the basic unit of the hexagonal prism according to the present disclosure;

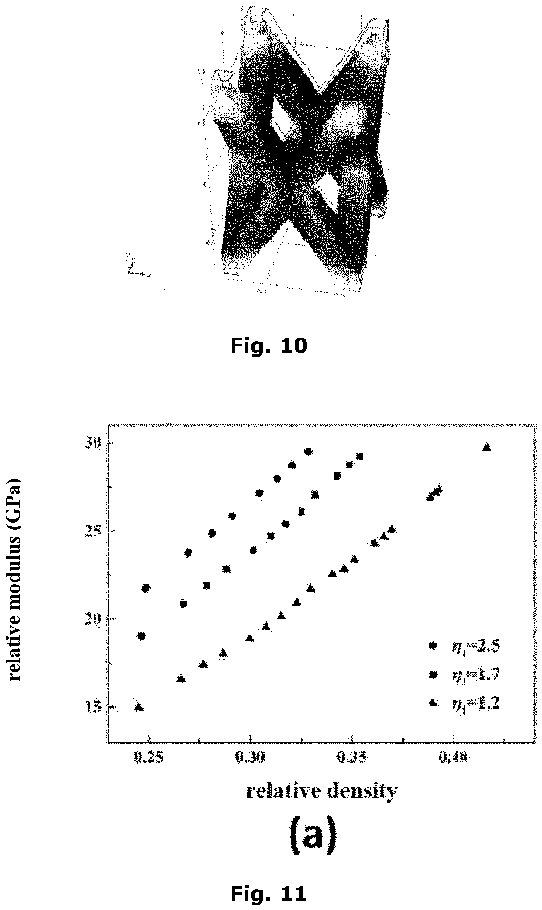

Figure 10    is the finite element simulation analysis process chart of the basic unit of the quadrangular prism according to the present disclosure;

Figure 11    is the state diagram of the change of the modulus of the hexagonal prism with the structural parameters.

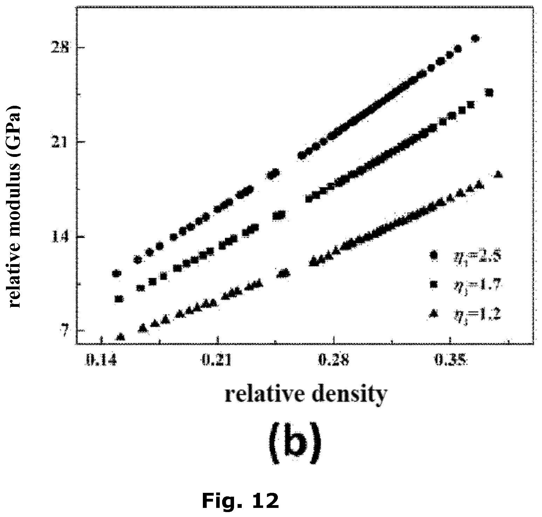

Figure 12    is the state diagram of the change of the modulus of the quadrangular prism with the structural parameters.

Meaning of the reference indicia of the accompanying drawings:

$r$    is the inscribed circle radius of the cross section of the central interconnected pore;

$t$    is the thickness or the equivalent circle diameter of the cross section of the rib of the "X-type" framework;

$l$    is the length of the rib of the "X-type" framework;

$c$    is the height of the quadrangular prism or the hexagonal prism;

$a$    is the base length of the quadrangular prism or the hexagonal prism;

$\eta_1$    is the ratio of the height c to the base length a of the quadrangular prism or the hexagonal prism.

**Specific Mode for Carrying Out the Invention**

The present disclosure will be illustrated hereinafter detailedly with reference to the following embodiments and examples.

The specific embodiments for carrying out the invention are described as follows.

A medical implant porous scaffold structure having low modulus, wherein said structure is formed by multiple basic units superposed sequentially along the three-dimensional directions in three-dimensional space, each of the basic units is composed of a quadrangular prism or hexagonal prism having central interconnected pores encircled by four or six side walls, each of the side walls is composed by a "X-type" frame structure formed by two crossed ribs, and the central interconnected pores of the adjacent basic units arranged along the axis direction of the quadrangular prism or the hexagonal prism are interconnected to each other.

The rib of the prism may have a cross section in the shape of solid circle, solid oval, solid polygon, hollow circular ring, hollow oval ring or hollow polygonal ring.

In order to meet the bio-functional requirements of the support materials, the inscribed circle radius $r$ of the cross section of the central interconnected pore is 150 μm to 750 μm.

The ratio of the equivalent circle diameter of the cross section of the ribs to the length of the rib is 0.1 to 0.5; the ratio of the height of the quadrangular prism or the hexagonal prism to the base length of the side wall of the quadrangular prism or the hexagonal prism is 1.0 to 2.5.

For the basic unit of the hexagonal prism, its structural feature of the overall shape can be determined by the ratio of $\eta_1$ of the height c to the base length $a$ of the hexagonal prism ($\eta_1=c/a$); the relative density of the basic unit of the hexagonal prism can be determined by the ratio of $\eta_2$ of the thickness $t$ of the rib of the "X-type" framework (or the equivalent circle diameter of the cross section) to the length $l$ of the rib of the "X-type" framework ($\eta_2=t/l$).

For the basic unit of the quadrangular prism, its structural feature of the overall shape can be determined by the ratio of $\eta_1$ of the height c to the base length a of the quadrangular prism ($\eta_1=c/a$); the relative density of the basic unit of the quadrangular prism can be determined by the ratio of $\eta_2$ of the thickness $t$ of the rib of the "X-type" framework (or the equivalent circle diameter of the cross section) to the length $l$ of the rib of the "X-type" framework ($\eta_2=t/l$).

The cross-sectional area of the central interconnected pore is larger than the cross-sectional area of the pores of the "X-type" framework structure in the side wall.

The whole structure of the scaffold structure can be calculated by finite element method. Firstly, establishing the geometric model of the scaffold (such as the "X-type" quadrangular prism or the "X-type" hexagonal prism, etc.) by a drawing software, and at the same time, setting the structure parameters ($r$, $\eta_1$ and $\eta_2$) of the scaffold structure; and then introducing a finite element analysis software (such as Ansys, Comsol or Abaqus, etc.), defining the parameters ($E$ and $v$, etc.) of the materials; setting the boundary conditions, loading conditions and dividing mesh; then carrying on the finite element calculation and analysis. According to the requirement of modulus lower than 30 GPa, if the whole modulus of the selected scaffold structure can meet the requirements, the selected scaffold structure solution would be established. Otherwise, the parameters ($r$, $\eta_1$ and $\eta_2$) of the scaffold structure should be reset, and calculated and judged in accordance with the above procedure, so as to obtain the materials of the scaffold structure and the parameters ranges meeting the conditions of low modulus.

**$\alpha$-Ti Example Group**

**Example 1**

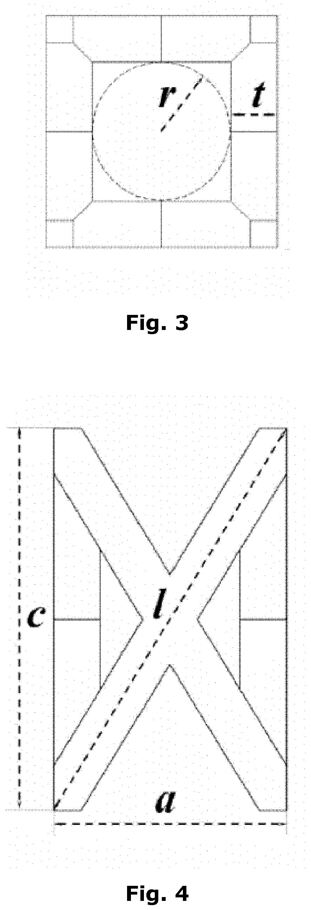

For the basic unit of the hexagonal prism, when $\alpha$-Ti (E=110 GPa, $v$=0.33) was selected as the implant materials, as shown in Figure 3, the finite element method can be used to calculate the relationship between the relative modulus of the scaffold materials and the relative density of the scaffold. The result showed that when $\eta_1$ was selected to range from 1.0 to 2.5, $\eta_2$ was selected to range from 0.10 to 0.50, and the inscribed circle radius $r$ of the interconnected pore was selected to range from 150 $\mu$m to 750 $\mu$m, the relative modulus of the scaffold materials could be less than 30 GPa, meeting the modulus range of human cortical bone.

**Example 2**

For the basic unit of the quadrangular prism, when $\alpha$-Ti ($E$=110 GPa, $v$=0.33) was selected as the implant materials, as shown in Figure 4, the finite element method can be used to calculate the relationship between the relative modulus of the scaffold materials and the relative density of the scaffold. The result showed that when $\eta_1$ was selected to range from 1.0 to 2.5, $\eta_2$ was selected to range from 0.1 to 0.35, and the inscribed circle radius r of the interconnected pore was selected to range from 150 $\mu$m to 750 $\mu$m, the relative modulus of the scaffold materials could be less than 30 GPa, meeting the modulus range of human cortical bone.

**Mg Example Group**

**Example 3**

For the basic unit of the hexagonal prism, when Mg ($E$=44 GPa, $v$=0.26) was selected as the implant materials, as shown in Figure 3, the finite element method can be used to calculate the relationship between the relative modulus of the scaffold materials and the relative density of the scaffold. The result showed that when $\eta_1$ was selected to range from 1 to 2.5, $\eta_2$ was selected to range from 0.1 to 0.5, and the inscribed circle radius $r$ of the interconnected pore was selected to range from 150 $\mu$m to 750 $\mu$m, the relative modulus of the scaffold materials could be less than 30 GPa, meeting the modulus range of human cortical bone.

**Example 4**

For the basic unit of the quadrangular prism, when Mg ($E$=44 GPa, $v$=0.26) was selected as the implant materials, as shown in Figure 4, the finite element method can be used to calculate the relationship between the relative modulus of the scaffold materials and the relative density of the scaffold. The result showed that when $\eta_1$ was selected to range from 1.2 to 2.5, $\eta_2$ was selected to range from 0.15 to 0.50, and the inscribed circle radius $r$ of the interconnected pore was selected to range from 150 $\mu$m to 750 $\mu$m, the relative modulus of the scaffold materials could be less than 30 GPa, meeting the modulus range of human cortical bone.

**Co-Cr-Mo Example Group**

**Example 5**

For the basic unit of the hexagonal prism, when Co-Cr-Mo ($E$=248 GPa, $v$=0.30) was selected as the implant materials, as shown in Figure 3, the finite element method can be used to calculate the relationship between the relative modulus of the scaffold materials and the relative density of the scaffold. The result showed that when $\eta_1$ was selected to range from 1.0 to 1.5, $\eta_2$ was selected to range from 0.10 to 0.25, and the inscribed circle radius $r$ of the interconnected pore was selected to range from 150 $\mu$m to 750 $\mu$m, the relative modulus of the scaffold materials could be less than 30 GPa, meeting the modulus range of human cortical bone.

**Example 6**

For the basic unit of the quadrangular prism, when Co-Cr-Mo ($E$=248 GPa, $v$=0.30) was selected as the implant materials, as shown in Figure 4, the finite element method can be used to calculate the relationship between the relative modulus of the scaffold materials and the relative density of the scaffold. The result showed that when $\eta_1$ was selected to range from 1.0 to 2.3, $\eta_2$ was selected to range from 0.10 to 0.45, and the inscribed circle radius $r$ of the interconnected pore was selected to range from 150 $\mu$m to 750 $\mu$m, the relative modulus of the scaffold materials could be less than 30 GPa, meeting the modulus range of human cortical bone.

The above specific embodiments were intended to detailedly explain the technical solution according to the disclosure. The present disclosure should not be limited to just the above embodiments. Any improvement or replacement according to the principles according to the disclosure should be included within the protection scope of the invention.

## Claims

1. A medical implant porous scaffold structure having low modulus, wherein said structure is formed by multiple basic units superposed sequentially along the three-dimensional directions in three-dimensional space, each of the basic units is composed of a quadrangular prism or hexagonal prism having central interconnected pores encircled by four or six side walls, and the central interconnected pores of the adjacent basic units arranged along the axis direction of the quadrangular prism or the hexagonal prism are interconnected to each other, **characterised in that** each of the side walls is composed by a X-type frame structure formed by two crossed ribs, and the ratio of the equivalent circle diameter of the cross section of the rib to the length of the rib is 0.1 to 0.5; the ratio of the height of the quadrangular prism or the hexagonal prism to the base length of the side wall of the quadrangular prism or the hexagonal prism is 1.0 to 2.5.

2. The medical implant porous scaffold structure having low modulus of claim 1, wherein the inscribed circle radius of the cross section of the central interconnected pore is 150 $\mu$m to 750 $\mu$m.

3. The medical implant porous scaffold structure having low modulus of claim 1 or 2, wherein the cross section area of the central interconnected pore is greater than the cross section area of the pore of the "X-type" framework structure in the sidewall.

## Patentansprüche

1. Eine poröse Gerüststruktur eines medizinischen Implantats mit niedrigen Modul, wobei die Struktur durch mehrere Grundeinheiten gebildet ist, die sequentiell entlang den dreidimensionalen Richtungen im dreidimensionalen Raum überlagert sind, wobei jede der Basiseinheiten aus einem viereckigen Prisma oder hexagonalen Prisma mit zentralen miteinander verbundenen Poren besteht, die von vier oder sechs Seitenwänden umgeben sind, und wobei die zentralen miteinander verbundenen Poren der benachbarten Grundeinheiten, die entlang der Achsenrichtung des viereckigen Prismas oder des hexagonalen Prismas angeordnet sind, miteinander verbunden sind, **dadurch gekennzeichnet, dass** jede der Seitenwände aus einer Rahmenstruktur vom X-Typ besteht, die aus zwei gekreuzten Rippen besteht, und das Verhältnis des äquivalenten Kreisdurchmessers des Querschnitts der Rippe zur Länge der Rippe 0,1 bis 0,5 beträgt; das Verhältnis der Höhe des viereckigen Prismas oder des hexagonalen Prismas zur Basislänge der Seitenwand des viereckigen Prismas oder des hexagonalen Prismas 1,0 bis 2,5 beträgt.

2. Poröse Gerüststruktur eines medizinischen Implantats mit niedrigem Modul nach Anspruch 1, wobei das Inkreisradius des Querschnitts der zentralen miteinander verbundenen Pore 150 $\mu$m bis 750 $\mu$m beträgt.

3. Poröse Gerüststruktur eines medizinischen Implantats mit niedrigem Modul nach Anspruch 1 oder 2, wobei die Querschnittsfläche der zentralen miteinander verbundenen Pore größer als die Querschnittsfläche der Pore der "X-Typ" Gerüststruktur in der Seitenwand ist.

## Revendications

1. Structure d'échafaudage poreux d'implant médical ayant un faible module, dans laquelle ladite structure est formée de multiples unités de base superposées séquentiellement le long des directions tridimensionnelles dans un espace tridimensionnel, chacune des unités de base étant composée d'un prisme quadrangulaire ou d'un prisme hexagonal avec pores interconnectés centraux entourés de quatre ou six parois latérales, et les pores interconnectés centraux des unités de base adjacentes agencées dans la direction de l'axe du prisme quadrangulaire ou du prisme hexagonal sont interconnectés les uns aux autres, **caractérisé en ce que** chacune des parois latérales est composée d'une structure de cadre de type X formée de deux nervures croisées, et le rapport du diamètre de cercle équivalent de la section transversale de la nervure à la longueur de la nervure est compris entre 0.1 et 0.5; le rapport de la hauteur du prisme quadrangulaire ou du prisme hexagonal à la longueur de base de la paroi latérale du prisme quadrangulaire ou du prisme hexagonal est compris entre 1.0 et 2.5.

2. Structure d'échafaudage poreux d'implant médical ayant un faible module selon la revendication 1, dans laquelle le rayon de cercle inscrit de la section transversale du pore interconnecté central est compris entre 150 et 750 $\mu$m.

**3.** Structure d'échafaudage poreux d'implant médical ayant un faible module selon la revendication 1 ou 2, dans laquelle la surface de section transversale du pore interconnecté central est supérieure à la surface de section transversale du pore de la structure de cadre de "type X" dans la paroi latérale.

**(a)**

**Fig. 1**

**(b)**

**Fig. 2**

**Fig. 3**

**Fig. 4**

establishing the
geometric model

↓

setting the structure
parameters of the scaffold
structure

↓

defining the parameters of the
materials

↓

setting the boundary conditions and
loading conditions

↓

dividing mesh

↓

calculation and analysis

↓

if meet the
modulus
requirement                    No

Yes

↓

mode solution
established

**Fig. 5**

**Fig. 6**

**Fig. 7**

**Fig. 8**

**Fig. 9**

**Fig. 10**

**relative modulus (GPa)**

**relative density**

**(a)**

**Fig. 11**

**(b)**

**Fig. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009048314 A